# EUROPEAN PATENT SPECIFICATION

(11) **EP 1 859 691 B1**
(45) Date of publication and mention of the grant of the patent: **08.07.2009**
(21) Application number: 07107871.1
(22) Date of filing: 10.05.2007
(51) Int. Cl.: A23L 1/30, A23L 2/38, A23F 3/00, A61K 36/886

(54) **Method for processing parts of aloe to obtain a base for preparing a beverage**
Verfahren zur Verarbeitung von Teilen von Aloe zur Gewinnung einer Basis zur Herstellung eines Getränks
Procédé pour le traitement de parties d'aloe pour obtenir une base de préparation d'une boisson

(30) Priority: 24.05.2006 IT BO20060394
(43) Date of publication of application: 28.11.2007
(73) Proprietor: Arini, Giuseppe, 40012 Calderara di Reno BO (IT)
(72) Inventor: Arini, Giuseppe, 40012 Calderara di Reno BO (IT)
(74) Representative: Modiano, Micaela Nadia

(56) References cited:
- WO-A-95/10199
- WO-A-20/05112962
- HE Q ET AL: "Quality and safety assurance in the processing of aloe vera gel juice" FOOD CONTROL, BUTTERWORTH, LONDON, GB, vol. 16, no. 2, February 2005 (2005-02), pages 95-104, XP004546841 ISSN: 0956-7135
- DATABASE BIOSIS [Online] BIOSCIENCES INFORMATION SERVICE, PHILADELPHIA, PA, US; 25 April 2002 (2002-04-25), FURUKAWA FUMIO ET AL: "Chemopreventive effects of Aloe arborescens on N-nitrosobis(2-oxopropyl)amine-induced pancreatic carcinogenesis in hamsters" XP002439527 Database accession no. PREV200200339637 & CANCER LETTERS, vol. 178, no. 2, 25 April 2002 (2002-04-25), pages 117-122, ISSN: 0304-3835

## Description

The present invention relates to a method for processing parts of aloe to obtain a base for preparing a beverage.

By using plants, it is possible to obtain many beverages which differ from one another in their properties, chemical-physical and organoleptic characteristics, and preparation method.

Depending on whether one begins with the leaves, stalks, flowers or fruits, on whether they are fresh and/or preserved (for example dried), and on the base liquid and its chemical-physical characteristics at the moment of contact with the parts of the plant, the resulting beverage may in fact be of any kind.

The choice of the preparation method depends first of all on the active ingredient to be obtained from the herbs. Essential oils are highly volatile and would evaporate if boiled. Accordingly, aromatic herbs are generally prepared in infusion, like tea, leaving them to stand in boiling water for five to thirty minutes. This is the case of mint, lemon-balm, vervain, elder, mugweed, thyme, absinthe, et cetera.

If the active ingredients of interest are instead mucilages, tannins, bitter oils or other less volatile elements, they can be obtained better by boiling the herbs together with the water, generally for a time which increases with woodiness (barks, roots, hard seeds and non-aromatic leaves, such as mallow). Further optimization can be achieved, in these cases, by leaving the herbs to soak in cold water for 2-3 to 12 hours prior to boiling, so that they soften and release their ingredients more easily.

A vast number of infusions, extracts and/or decoctions based on a plant or on a mixture of different plants, which combine a pleasant taste with one or more substantially curative properties, are commercially available.

Consider, for example, the plurality of infusions, extracts and/or decoctions described as cleansing, which have a draining and diuretic action (for example an artichoke infusion), or relaxing ones (for example chamomile and/or valerian) and stimulants (one among many: coffee).

These beverages predominantly perform one function, and therefore in order to obtain simultaneously the benefits of a plurality of herbal substances it is necessary to create blends.

Beverages obtained with blends of various substances generally have a weaker effect (in the same amount of material immersed in the base liquid, the concentration of each individual component is of course reduced with respect to the case of a preparation which uses a single substance).

To ensure the simultaneous effects of a plurality of substances to the user, the user would have to drink large quantities of beverages derived from each of said substances: this is obviously a possibility which is difficult to apply.

The aim of the present invention is to provide a method for processing parts of aloe to obtain a base for preparing a beverage having evident and heterogeneous beneficial properties for the user.

Within this aim, an object of the present invention is to provide a digestive beverage which is adapted to regulate intestinal transit, reduce meteorism and balance the intestinal bacterial flora.

Another object of the present invention is to provide an antibacterial and antimycotic beverage.

Another object of the present invention is to provide a beverage which helps to reduce the level of LDL cholesterol in blood.

Another object of the present invention is to provide a beverage which helps to balance the blood sugar level.

Another object is to provide a beverage which helps to stimulate immune defenses.

Another object of the present invention is to provide a beverage which helps to reduce the ailments of andropause and menopause.

Another object of the present invention is to provide a beverage which, in combination with an appropriate diet and with physical exercise, helps to lose weight.

Another object of the present invention is to provide a method for processing parts of aloe to obtain a base for preparing a beverage having low costs which is relatively simple to provide in practice and is safe in application.

This aim and these and other objects which will become better apparent hereinafter, are achieved by the present method for processing parts of aloe to obtain a base for preparing a beverage which consists in drying the parts of a plant known as Aloe, washing the dried parts, sterilizing the parts, freeze-drying the parts, shredding the parts and milling the parts.

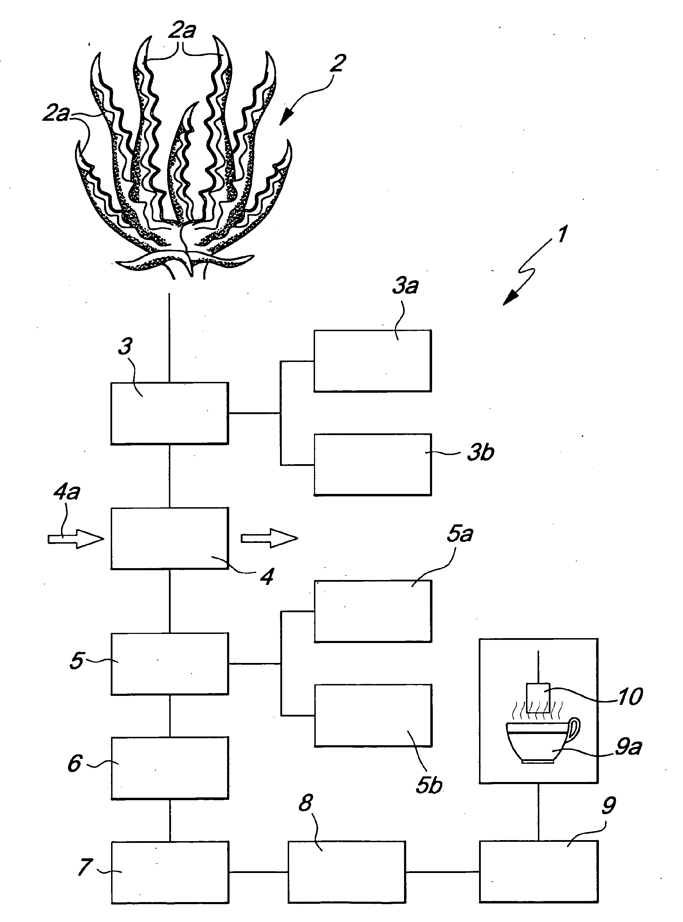

Further characteristics and advantages of the invention will become better apparent from the following detailed description of a preferred but not exclusive embodiment of a method for processing parts of aloe to obtain a base for preparing a beverage, illustrated by way of nonlimiting example in the accompanying drawings, wherein the sole Figure is a schematic view of a method for processing parts of aloe to obtain a beverage according to the invention.

With reference to the figure, the reference numeral 1 generally designates a method for processing parts of aloe to obtain a base for preparing a beverage.

The method 1 uses as raw material plants of Aloe 2, particularly the plant variety of Aloe known as Aloe Vera or Aloe Barbadensis.

This plant is widely used in the herbal field and in particular the secretion which can be extracted from its leaves (which have a fleshy appearance) and has the appearance of a gel is used. This substance is used universally for its positive effects in particular on damaged skin (in case of abrasions, bums, wounds and/or irritations of any kind).

The method 1 according to the invention instead uses the leaves 2a of Aloe Vera 2 instead of the gel secreted by said leaves as is instead known in the herbal field.

The method 1 provides for a first step 3 for drying the leaves 2a.

Drying 3 can be performed both artificially 3a, by using ovens or environments at high temperature with low and controlled humidity, and naturally 3b, by exposing the cut leaves 2a to the sun for a time sufficient to eliminate their water content.

Once drying 3 is complete, it is necessary to wash 4 the dried leaves 2a: the washing operation 4 can be performed conveniently by immersion in water 4a or by the passage of a stream of water 4a (for example showers and/or successive sprays from all directions).

One then proceeds with sterilization 5 of the leaves 2a: this step can be performed according to a method which is known universally as UHT 5a, which entails bringing the leaves 2a, for a minimum time of one second, to a temperature of at least 135°C; as an alternative, it is possible to perform a sterilization 5 by means of an autoclave 5b according to production requirements.

Once the leaves 2a have been sterilized 5, they must be freeze-dried 6 according to any one of the standard techniques, depending on production requirements.

The leaves 2a that have been freeze-dried 6 must then undergo homogenization processes: first of all, shredding 7 must be performed, followed by milling 8.

The coarse-grained powders obtained at the end of the entire process 1 must be substantially immersed 9 in a liquid to prepare the beverage 9a.

In general, the powders can be released into the liquid: in this case, it is necessary to filter the beverage 9a to eliminate the residues of the powder obtained by processing the leaves 2a.

According to some embodiments of higher practical interest, it is possible to package the powders in appropriate bags 10 made of permeable material (for example a fabric or microfiber or paper-like material having adequate mechanical strength) according to a preset dosage (for example the dose required to prepare a cup of the beverage 9a).

The liquid with which the beverage 9 according to the invention is generally obtained is water: it is possible to prepare infusions, decoctions, tisanes and/or other similar preparations.

It should be noted that it is also possible to provide alcoholic solutions starting from the coarse-grained powders obtained at the end of the method 1.

It has thus been shown that the invention achieves the proposed aim and objects.

All the details may further be replaced with other technically equivalent elements.

In the exemplary embodiments shown, individual characteristics, given in relation to specific examples, may actually be interchanged with other different characteristics that exist in other exemplary embodiments.

Moreover, it is noted that anything found to be already known during the patenting process is understood not to be claimed and to be the subject of a disclaimer.

In practice, the materials used, as well as the shapes and dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A method for processing parts of aloe (2) to obtain a base for preparing a beverage, comprising the steps of:
- drying (3) parts of a plant known as Aloe (2),
- washing (4) the dried parts,
- sterilizing (5) the parts,
- freeze-drying (6) the parts,
- shredding (7) the parts,
- milling (8) the parts.

2. The method according to claim 1, **characterized in that** the parts of the plant (2) are the leaves (2a).

3. The method according to claim 1, **characterized in that** the plant variety of Aloe (2) is Aloe Vera, also known as Aloe Barbadensis.

4. The method according to claim 1, **characterized in that** said washing (4) is performed with potable water.

5. The method according to claim 1, **characterized in that** said sterilization (5) is of the high-temperature type, a technique known as UHT sterilization (5a).

6. The method according to claim 1, **characterized in that** said sterilization (5) provides for introducing the parts in an autoclave (5b).

7. The method according to claim 1, **characterized in that** in order to obtain the beverage (9a) it is necessary to immerse (9) the milled parts in a liquid.

8. The method according to claim 7, **characterized in that** said milled parts are subjected to immersion (9) in generally hot water.

9. A beverage of the type obtainable by immersing plant derivatives in water, **characterized in that** said plant derivatives are the leaves (2a) of a plant (2) known as Aloe, in the form of coarse powder.

10. The beverage according to claim 9, **characterized in that** the plant variety of Aloe (2) is the one known as Aloe Vera, also known as Aloe Barbadensis.

11. The beverage according to claim 9, **characterized in that** a bag (10) of permeable material contains a predefined dose of said plant derivatives, said bag (10) and its content being immersible in hot water.

## Patentansprüche

1. Verfahren zur Verarbeitung von Teilen von Aloe (2), um eine Grundlage zur Herstellung eines Getränks zu erhalten, beinhaltend folgende Schritte:
- Trocknen (3) von Teilen einer Pflanze, die als Aloe bekannt ist (2),
- Waschen (4) der getrockneten Teile,
- Sterilisieren (5) der Teile,
- Gefriertrocknen (6) der Teile,
- Schreddern (7) der Teile
- Zermahlen (8) der Teile.

2. Verfahren nach Anspruch 1, **dadurch gekennzeichnet, dass** die Teile der Pflanze (2) die Blätter sind (2a).

3. Verfahren nach Anspruch 1, **dadurch gekennzeichnet, dass** die Pflanzensorte von Aloe (2) Aloe Vera ist, auch bekannt als Aloe Barbadensis.

4. Verfahren nach Anspruch 1, **dadurch gekennzeichnet, dass** das Waschen (4) mit Trinkwasser erfolgt.

5. Verfahren nach Anspruch 1, **dadurch gekennzeichnet, dass** die Sterilisation (5) vom Hoch-Temperatur Typ ist, eine Technik, die als UHT Sterilisation (5a) bekannt ist.

6. Verfahren nach Anspruch 1, **dadurch gekennzeichnet, dass** zur Sterilisation (5) vorgesehen ist, die Teile in einen Autoklaven einzubringen (5b).

7. Verfahren nach Anspruch 1, **dadurch gekennzeichnet, dass** um das Getränk (9a) zu erhalten, es notwendig ist, die zermahlenen Teile in eine Flüssigkeit zu tauchen (9).

8. Verfahren nach Anspruch 7**, dadurch gekennzeichnet, dass** die zermahlenen Teile in generell heißes Wasser getaucht (9) werden.

9. Getränk vom Typ, welcher **dadurch** erhalten werden kann, dass Pflanzenderivate in Wasser getaucht werden, **dadurch gekennzeichnet, dass** diese Pflanzenderivate die Blätter (2a) einer Pflanze (2), die als Aloe bekannt ist, in Form von Grobpulver sind.

10. Getränk nach Anspruch 9, **dadurch gekennzeichnet, dass** die Pflanzensorte von Aloe (2) diejenige ist, die als Aloe Vera, auch als Aloe Barbadensis bekannt ist.

11. Getränk nach Anspruch 9, **dadurch gekennzeichnet, dass** ein Beutel (10) aus durchlässigem Material eine bestimmte Menge dieser Pflanzenderivate enthält, wobei dieser Beutel (10) und sein Inhalt in heißes Wasser getaucht werden kann.

## Revendications

1. Procédé de traitement de parties d'aloès (2) pour obtenir une base de préparation d'une boisson, comprenant les étapes consistant à :
- sécher (3) les parties d'une plante connue sous le nom d'aloès (2),
- laver (4) les parties séchées,
- stériliser (5) les parties,
- lyophiliser (6) les parties,
- déchiqueter (7) les parties,
- broyer (8) les parties.

2. Procédé selon la revendication 1, **caractérisé en ce que** les parties de la plante (2) sont les feuilles (2a).

3. Procédé selon la revendication 1, **caractérisé en ce que** la variété de l'aloès (2) est l'Aloe Vera, également connue sous le nom d'Aloe Barbadensis.

4. Procédé selon la revendication 1, **caractérisé en ce que** ledit lavage (4) est effectué avec de l'eau potable.

5. Procédé selon la revendication 1, **caractérisé en ce que** ladite stérilisation (5) est de type à haute température, une technique connue sous le nom de stérilisation UHT (5a).

6. Procédé selon la revendication 1, **caractérisé en ce que** ladite stérilisation (5) prévoit l'introduction des parties dans un autoclave (5b).

7. Procédé selon la revendication 1, **caractérisé en ce que**, afin de produire la boisson (9a), il est nécessaire d'immerger (9) les parties broyées dans un liquide.

8. Procédé selon la revendication 7, **caractérisé en ce que** lesdites parties broyées sont soumises à une immersion (9) dans de l'eau généralement chaude.

9. Boisson du type pouvant être obtenue par immersion de dérivés végétaux dans de l'eau, **caractérisée en ce que** lesdits dérivés végétaux sont les feuilles (2a) d'une plante (2) connue sous le nom d'aloès, sous la forme d'une poudre grossière.

10. Boisson selon la revendication 9, **caractérisée en ce que** la variété d'aloès (2) est celle connue sous le nom d'Aloe Vera, également connue sous le nom d'Aloe Barbadensis.

11. Boisson selon la revendication 9, **caractérisée en ce qu'**un sachet (10) constitué d'un matériau perméable contient une dose prédéfinie desdits dérivés végétaux, ledit sachet (10) et son contenu pouvant être immergés dans de l'eau chaude.
